# EUROPEAN PATENT SPECIFICATION

(11) **EP 2 319 557 B1**
(45) Date of publication and mention of the grant of the patent: **28.08.2019**
(21) Application number: 10184284.7
(22) Date of filing: 20.02.2006
(51) Int. Cl.: A61M 5/14, A61M 1/36, A61M 5/36, A61M 5/168, A61M 39/22, A61M 39/24

(54) **Pressure activated IV set with drip chamber having a lateral access and a pressure valve**
Druckaktiviertes IV Set mit Tropfenkammer mit seitlichem Zugang und mit Druckventil
IV set activé par pression comprenant un goutte à goutte ayant un accès latéral et une valve activée par pression

(30) Priority: 18.02.2005 US 654705 P; 03.10.2005 US 242329; 03.10.2005 US 242353
(43) Date of publication of application: 11.05.2011
(62) Divisional of application: 06250887.4
(73) Proprietor: Becton Dickinson and Company, Franklin Lakes, NJ 07417-1880 (US)
(72) Inventor: Adams, Chad, Cedar Hills, UT 84062 (US)
(74) Representative: Pawlyn, Anthony Neil

(56) References cited:
- EP-A2- 1 559 442
- WO-A-03/028525
- WO-A-2004/064904
- DE-A1- 4 142 625
- US-A- 3 756 233
- US-A- 3 931 818
- US-A- 4 013 072
- US-A- 4 269 222
- US-A1- 2004 254 542
- US-B1- 6 224 578
- US-B1- 6 336 916

## Description

### CROSS-REFERENCED RELATED APPLICATIONS

This application claims the benefit of U.S. Provisional Application No. 60/654,705, filed 2/18/2005.

### BACKGROUND OF THE INVENTION

This invention relates generally to tubing sets used in the administration of liquids to a patient that are commonly referred to as intravascular ("IV") sets and more particularly concerns pressure activated IV sets. An IV set according to the invention is used broadly herein to include tubing sets used in the arterial, intravenous, intravascular, peritoneal, and non-vascular administration of liquid into a patient. Of course, one of skill in the art may use an IV set to administer liquids to other locations within a patient's body than those listed.

Generally, an IV set includes tubing for connecting a patient to a source of liquid. Additionally, an IV set may include a drip chamber connected to the tubing, which is used by a nurse, physician, or other attendant to determine the flow rate of liquid through the IV set. The drip chamber is shaped to encourage liquid entering the drip chamber to form droplets that fall toward the bottom of the drip chamber. By counting each droplet over a period of time, the flow rate of liquid is able to be discerned.

When an IV set is prepared for use with a patient, a clamp on the tubing is manually closed to prevent liquid from moving from the drip chamber through the tubing. The IV set may then be attached to a source of liquid, such as an IV bag or bottle. Once attached, the drip chamber is squeezed to force air out of the drip chamber which is replaced by liquid from the IV bag or bottle.

Once the height of the liquid in the drip chamber is sufficient for operation of the IV set, the clamp is manually opened to allow liquid to flow through and replace air in the tubing of the IV set. However, as the liquid flows through the tubing air is frequently trapped in the tubing. For example, the flow of the liquid through the tubing of the IV set may be turbulent and can entrap air as the boundary layer between the liquid and the tubing is sheared. Additionally, if the liquid level is too low, a bubble ladder may form as air is intermittently sucked from the drip chamber into the tubing.

Once the liquid is distributed throughout the tubing of the IV set, it is a generally good practice to remove entrapped air from the IV set. While this concern is critical when accessing arterial blood, it is also a concern when accessing the venous side. Specifically, if air bubbles are allowed to enter a patient's blood stream while receiving the intravenous administration of liquids, the air bubbles can form an air embolism and cause serious injury to a patient.

To remove air bubbles from the IV set, liquid from the IV bag or bottle is allowed to flow through the tubing while an attendant taps the tubing to encourage the air bubbles out the end of the IV set. As the liquid is allowed to flow out of the IV set to clear air bubbles from the tubing, the liquid is generally allowed to flow into a waste basket or other receptacle. During this procedure the end of the tubing may contact the waste basket or be touched by the attendant and thus, become contaminated.

Once the entrapped air is removed, the IV set is ready to be connected to a patient. This process of preparing an IV set for connection to a patient is commonly referred to as "priming" an IV set.

If there is a delay between priming the IV set and connecting it to a patient, a solid cap may be attached to the end of the IV set to completely close and protect the end of the IV set from contamination. The cap may then be removed when the patient is ready to have the IV set connected.

In administering medicines to a patient, the IV set often provides access for a nurse or physician. Currently available IV sets generally include a Y or T connector disposed along the tubing. These Y and T connectors may be used to piggyback a second IV set or to add medication through a syringe.

A short coming of these Y and T connectors is that they do not provide control over the rate at which liquid enters the tubing at the Y and T connectors. Additionally, medicines and liquids administered through the Y or T connector may not mix thoroughly with the liquid already traveling in the tubing of the IV set, which may be a concern in some applications.

Accordingly, a need exists for an IV set that is self-priming, and which does not require constant attention and supervision. A need also exists for an IV set that is self-leveling to ensure that an operable liquid height is maintained in the drip chamber. Additionally, a need exists for an IV set that prevents air from entering the tubing when the IV bag or bottle runs dry.

A need also exists for an IV set that provides better control over the liquid height within the drip chamber in order to better control the priming and use of the IV set. A need also exists for an IV set that includes safe guards that prevent a drip chamber from being over filled with liquid. Additionally, a need exists for an IV set that permits better control and mixing of liquids entering the IV set from a piggybacked IV set or syringe.

### BRIEF SUMMARY OF THE INVENTION

The present invention provides a pressure activated IV set for use in intravenous administration of liquids that may be self-priming and self-leveling. The IV set may include a coupling for connecting it to a source of liquid. The coupling may be a Luer fitting, a spike, or other coupling known in the art. A drip chamber may be connected to the coupling for determining the flow rate of liquid through the IV set. Typically, IV sets are gravity fed so that the drip chamber may include an inlet orifice disposed in a top end and an outlet orifice disposed in a bottom end of the drip chamber. A sidewall may extend between the top end and the bottom end.

A pressure activated valve may be connected to the outlet orifice of the drip chamber that may control the flow of liquid through the outlet orifice of the drip chamber. In some configurations, the pressure activated valve may abut or be integrally formed with the drip chamber. The pressure activated valve may operate so that when an operable liquid height in the drip chamber is reached, the pressure activated valve opens to permit liquid to exit the outlet orifice of the drip chamber. Additionally, when the liquid height falls below the operable liquid height, the pressure activated valve closes to prevent liquid from exiting the outlet orifice of the drip chamber.

The operable liquid height is the height of liquid from the sealing orifice to a height within the drip chamber. The operable liquid height may be approximately the height of liquid at which the drip chamber is normally filled when the IV set is ready to be connected to a patient. Thus, the operable liquid height may range from about 1/4 to about 2/3 the height of the drip chamber. Preferably, the operable liquid height may range from about 1/3 to about 1/2 the height of the drip chamber.

When priming the IV set, liquid may be unable to enter the drip chamber from a source of liquid as the pressure within the drip chamber increases. To release this pressure, the drip chamber may include an air vent. The air vent may be designed to prevent liquid from passing through the vent, while allowing air to exit the drip chamber. The air vent may also permit air to only exit and not to enter the drip chamber. Additionally, the air vent may be connected to a sidewall of the drip chamber proximate to the operable liquid height. This helps to maintain a desirable liquid level within the drip chamber, by preventing additional air from exiting the drip chamber once liquid reaches the air vent.

Referring back to the IV set, the IV set may include a tube having a first end and a second end that may be connected to the pressure activated valve. The first end may include a coupling, such as a Luer fitting to facilitate connecting the IV set to a patient.

An end plug may be connected to the coupling of the first end of the tube. The end plug may include an air vent designed to permit air to exit and prevent liquid from exiting. The end plug may help to prevent contamination of the IV set and may be removed and discarded when the IV set is to be connected to a patient.

The air vent of the end plug may be used to slow the rate at which air exits the IV set. By slowing the exit of air from the IV set, liquid flowing through the IV set is also slowed. Slow flowing liquid is less likely to entrap air in the IV set, which reduces the need to remove air bubbles from the IV set.

A filter may be used to permit air to exit or enter the drip chamber through an access orifice, while preventing liquid from exiting the drip chamber. A valve may be disposed to control the flow of fluid through the filter. The filter may be positioned proximate to an operable liquid height in the drip chamber. The filter may include a screen made of a hydrophobic material that repels water and has a small pore size or a plurality of small holes that permit air to exit the drip chamber while restricting liquids from exiting the drip chamber.

The IV set of the invention may be primed by connecting the coupling of the IV set to a container of liquid, opening the valve connected to the drip chamber to permit air to exit the drip chamber, and filling the drip chamber with liquid. As the drip chamber fills with liquid, the valve may be adjusted to alter the flow rate of liquid into the drip chamber. Once the drip chamber is filled to an operable liquid height, the valve may be closed to prevent the exiting of air through the access orifice and to prevent the drip chamber from being filled to greater than the operable liquid height.

To retard the rate at which the liquid flows through tubing of the IV set during priming, and in order to avoid trapping air in the tubing, the IV set may include a tube attachment device. During priming of the IV set, the tube attachment device may be used to dispose the end of the tube proximate to the drip chamber. Disposing the end of the tube proximate to the drip chamber permits the hydrostatic pressure of the liquid to slow the flow of liquid through the tube and helps to prevent liquid from inadvertently rushing out of the end of the tube as the IV set is primed.

Where the IV set also includes a filter, air from the drip chamber may be passed through the filter during priming of the IV set. The filter may also be used to inhibit the flow of liquid through the filter. Thus, if the filter is positioned at or proximate to an operable liquid height and the valve is inadvertently left open, the liquid in the drip chamber will not be able to exit the drip chamber through the access orifice. Additionally, once the access orifice is covered with liquid, air will not be able to exit the drip chamber, which forces the level of liquid in the drip chamber to maintain a level at about the top of the access orifice. Thus, the filter may act as an overfill safety device.

In configurations of the IV set that include a pressure activated valve, the method of priming the IV set may also include the step of opening the pressure activated valve to permit the flow of liquid through the outlet orifice of the drip chamber when the pressure of the liquid within the drip chamber is greater than the force of a biasing mechanism of the pressure activated valve. The method may also include the step of closing the pressure activated valve to prevent the flow of liquid through the outlet orifice of the drip chamber when the pressure of the liquid within the drip chamber is less than the force of a biasing mechanism of the pressure activated valve.

In configurations of the IV set that include an access port connected to the access orifice, the access port may be used to connect a second IV set to the access orifice. The access port may also be used to connect a removable filter to the access orifice.

These and other features and advantages of the present invention will become more fully apparent from the following description and appended claims, or may be learned by the practice of the invention as set forth hereinafter.

### STATEMENTS OF INVENTION

According to a first aspect the invention provides an IV set comprising:
a drip chamber having an outlet orifice; and
a pressure activated valve disposed proximate the outlet orifice of the drip chamber, the pressure activated valve comprising:
   a sealing orifice;
   a valve; and
   a biasing mechanism that biases the valve against the sealing orifice with a force less than the pressure head between the sealing orifice and an operable liquid height in the drip chamber to control the flow of liquid through the pressure activated valve.

The IV set may further comprise an air vent connected to the drip chamber. The air vent may be connected to a sidewall of the drip chamber proximate to the operable liquid height. The biasing mechanism may comprise a spring. The biasing mechanism may comprise a magnet. The biasing mechanism may comprise a flexible arm.

The IV set may further comprise a tube having a first end and a second end connected to the pressure activated valve, wherein the IV set further comprises a tube attachment device for disposing the first end of the tube proximate to the drip chamber.

The IV set may further comprise a tube having a first end and a second end connected to the pressure activated valve, wherein the IV set further comprises an end plug removably attached to the first end of the tube, wherein the end plug comprises an air vent

According to a second aspect the invention provides an IV set comprising:
a drip chamber having an outlet orifice; and
a pressure activated valve disposed proximate the outlet orifice of the drip chamber, the pressure activated valve comprising:
   a sealing orifice;
   a valve for closing the sealing orifice; and
   a means for biasing the valve against the sealing orifice with a force less than the pressure head between the seating orifice and an operable liquid height in the drip chamber to control the flow of liquid through the pressure activated valve.

The IV set may further comprise an air vent connected to the drip chamber. The air vent may be attached to a sidewall of the drip chamber proximate to the operable liquid height.

The IV set may further comprise a tube having a first end and a second end connected to the pressure activated valve, wherein the IV set further comprises a tube attachment device for disposing the first end of the tube proximate to the drip chamber.

The IV set may further comprise a tube having a first end and a second end connected to the pressure activated valve, wherein the IV set further comprises an end plug removably attached to the first end of the tube, wherein the end plug comprises an air vent.

According to a third aspect the invention provides an IV set comprising:
a drip chamber comprising an access orifice, a top end having an inlet orifice, a bottom end having an outlet orifice, and a sidewall extending between the top end and the bottom end,
wherein the access orifice is disposed in the sidewall;
a coupling for connecting the IV set to a source of liquid;
a valve connected to the drip chamber for selectively controlling access to the drip chamber through the access orifice; and
a tube connected to the outlet orifice of the drip chamber.

The IV set may further comprise a filter connected to the access orifice of the drip chamber for permitting air to flow through the filter while restricting the flow of liquid. The IV set may further comprise an access port connected to the access orifice.

The valve may be disposed to control the flow of fluid through the access port. The access port may comprise a septum. The access orifice may be disposed in the sidewall at or proximate to an operable liquid height.

The IV set may further comprise a pressure activated valve disposed to control the flow of liquid through the outlet orifice of the drip chamber.

The tube may comprise a first end connected to the drip chamber and a second end, wherein the IV set further comprises a tube attachment device for disposing the second end of the tube proximate to the drip chamber.

The IV set may further comprise a manifold connected to the access orifice.

According to a fourth aspect the invention provides an IV set comprising:
a drip chamber comprising an access orifice, a top end having an inlet orifice, a bottom end having an outlet orifice, and a sidewall extending between the top end and the bottom end,
wherein the access orifice is disposed in the sidewall;
a coupling for connecting the IV set to a source of liquid;
a means for selectively controlling access to the drip chamber through the access orifice, wherein the control means is connected to the drip chamber; and
a tube in liquid communication with the outlet orifice of the drip chamber.

The IV set may further comprise a filter connected to the access orifice of the drip chamber for permitting air to flow through the filter while restricting the flow of liquid. The IV set may further comprise an access port connected to the access orifice of the drip chamber. The access port may comprise a septum.

The venting orifice may be connected to the sidewall at or proximate to an operable liquid height. A manifold may be connected to the access orifice.

### BRIEF DESCRIPTION OF THE SEVERAL VIEWS OF THE DRAWINGS

In order that the manner in which the above-recited and other features and advantages of the invention are obtained will be readily understood, a more particular description of the invention briefly described above will be rendered by reference to specific embodiments thereof which are illustrated in the appended drawings. Understanding that these drawings depict only typical embodiments of the invention and are not therefore to be considered to be limiting of its scope, the invention will be described and explained with additional specificity and detail through the use of the accompanying drawings in which:
Figure 1 is a perspective view of an IV set according to the invention connected to an IV bag;
Figure 2 is a cross sectional view of a valve assembly connected to a drip chamber of an IV set for selectively controlling access to the drip chamber through the access orifice;
Figure 3 is a cross sectional view of another valve assembly connected to a drip chamber of an IV set;
Figure 4 is a cross sectional view of an alternative valve assembly connected to a drip chamber of an IV set according to the invention;
Figure 5 is a side elevation view of an IV set piggybacked on an IV set according to the invention.
Figure 6 is a perspective view of an IV set according to another embodiment of the invention connected to an IV bag;
Figure 7 is a partially cut away side view of another IV set according to the invention connected to an IV bottle;
Figure 8 is a partially cut away side view of an alternative IV set according to the invention;
Figure 9 is a partially cut away side view of yet another IV set according to the invention; and
Figure 10 is a partially cut away side view of yet another IV set according to the invention.

### DETAILED DESCRIPTION OF THE INVENTION

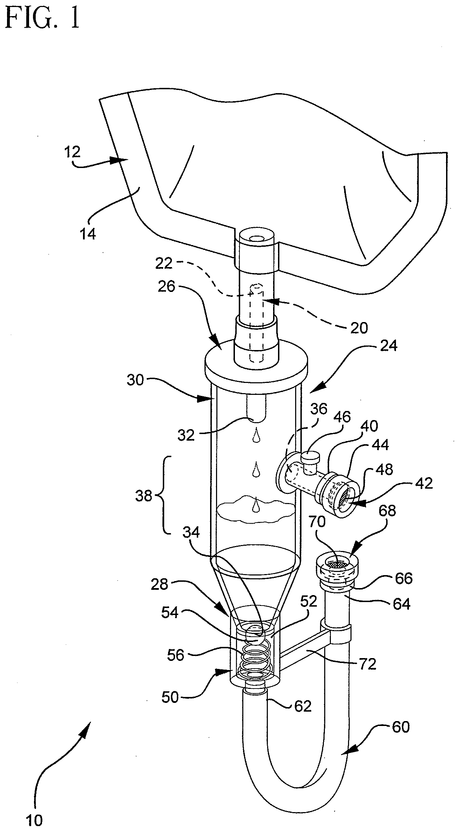

The presently preferred embodiments of the present invention will be best understood by reference to the drawings, wherein like parts are designated by like numerals throughout. It will be readily understood that the components of the present invention, as generally described and illustrated in the figures herein, could be arranged and designed in a wide variety of different configurations. Thus, the following more detailed description of the embodiments of the IV set of the present invention, as represented in Figures 1 through 10, is not intended to limit the scope of the invention, as claimed, but is merely representative of presently preferred embodiments of the invention.

Referring to Figure 1, a perspective view illustrates an IV set 10 according to the invention. As shown, the IV set 10 may be connected to a source of liquid 12, which in this configuration is an IV bag 14. Alternatively, the source of liquid 12 may be an IV bottle (not shown) or other container known in the art.

The IV set 10 may be connected to the IV bag 12 by a coupling 20 for connecting the IV set 10 to the IV bag 14. As shown, the coupling 20 may be a spike 22 for spiking the IV bag 14 and/or a threaded coupling (not shown).

The IV set 10 may include a drip chamber 24 for determining the flow rate from the source of liquid 12. The drip chamber 24 may include a top end 26, a bottom end 28, and a sidewall 30 extending between the top end 26 and the bottom end 28. The top end 26 may include an inlet orifice 32 that may be shaped and sized to encourage liquid entering the drip chamber 24 to form droplets, facilitating the determination of the liquid's flow rate. The bottom end 28 may also include an outlet orifice 34 that permits liquid to exit the drip chamber 24.

The drip chamber 24 may also include an access orifice 36. The access orifice 36 may be positioned in the top end 26 or in the sidewall 30. In some configurations, the access orifice 36 may be positioned at or near an operable liquid height 38 of the drip chamber 24. The operable liquid height 38 of the drip chamber 24 is deep enough so that air will not be sucked from the surface of the liquid into the outlet orifice 34, yet shallow enough that each droplet that falls from the inlet orifice 32 may be discerned in order to determine the flow rate of liquid into the drip chamber 24. For example, the operable liquid height 38 may range from about 1/3 to about 2/3 full. However, the preferable operable liquid height 38 of drip chamber 24 may range from about 1/3 to about 1/2 full.

An access port 40 may be connected to the access orifice 36. The access port 40 may be used to removably connect the access orifice 36 to various devices 42, such as a cap 44, another IV set, a syringe, and other devices known in the art. As shown, the access port 40 may be a Luer fitting, known in the art.

A valve 46 may also be connected to the drip chamber 24 to selectively control access to the drip chamber 24 through the access orifice 36. The valve 46 may be a stop cock, a slide valve, a butterfly valve, or any other type of valve known in the art. The valve 46 may be opened to allow gravity to pull liquid from the source of liquid 12 into the drip chamber 24 without deforming the sidewall 30 of the drip chamber 24 to induce a vacuum. The valve 46 may remain open until the operable liquid height 38 is reached. When the valve 46 is closed, fluid may only enter and exit through the inlet orifice 32 and outlet orifice 34. Thus, when the valve 46 and the outlet orifice 34 are closed, the pressure within the drip chamber 24 may rise until the liquid is unable to enter the drip chamber 24 from the inlet orifice 32.

In configurations of the IV set 10 where the access port 40 and the valve 46 are connected to the access orifice 36, the valve 46 may be disposed to prevent fluid from the access port 40 from reaching the access orifice 36. For example, the valve 46 may be used to control the flow from another IV set connected to the access port 40 into the drip chamber 24.

Additionally, a filter 48 may be connected to the access orifice 36, permitting air to flow through the filter 48 while restricting the flow of liquid. Where the access orifice 36 is disposed at an operable liquid height 38, the filter 48 may be used as a fail safe to prevent the height of liquid within the drip chamber 24 from exceeding the operable liquid height 38 of the drip chamber 24. More specifically, once the filter 48 is covered by the liquid within the drip chamber 24, the liquid and air in the drip chamber 24 is prevented from exiting the access orifice 36. Thus, the pressure may rise within the drip chamber 24 and prevent liquid from entering the drip chamber 24 from the source of liquid 12.

The IV set 10 may also include a pressure activated valve 50 disposed to control the flow of liquid through the outlet orifice 34 of the drip chamber 24. The pressure activated valve 50 may include a passageway 52 extending from the outlet orifice 34, a valve head 54 for closing the passageway 52, and a biasing mechanism 56 that biases the valve head 54 toward closing the passageway 52. The biasing mechanism 56 applies a force to the valve head 54 that may be about equal to the pressure exerted against the valve head 54 by the liquid filling the drip chamber 24 to approximately the operable liquid height 38. Thus, the pressure activated valve 50 prevents liquid from exiting the outlet orifice 34 as the drip chamber 24 fills with liquid, and permits liquid to exit the outlet orifice 34 when the height of the liquid is about equal to or greater than the operable liquid height 38. When the height of the liquid drops below the operable liquid height 38, the valve head 54 closes the passageway 52 preventing liquid from exiting the outlet orifice 34.

Once the pressure activated valve 50 is opened by the pressure of the liquid in the drip chamber 24, the valve 46 may be closed to prevent air from entering the drip chamber and to permit the flow rate of the liquid entering the drip chamber 24 to be approximately equal to the flow rate of the liquid exiting the drip chamber 24.

A tube 60 may be connected to the outlet orifice 34 of the drip chamber 24 through the pressure activated valve 50. More specifically, the tube 60 may have a first end 62 connected to the pressure activated valve 50 and a second end 64 that may be disposed remotely from the first end 62. The second end may include a connector 66 for connecting the IV set 10 to a patient (not shown). The connector 66 may be a Luer fitting or some other connector known in the art.

An end cap 68 may be coupled to the connector 66. The end cap 68 may be solid or alternatively, may include a filter 70 that permits air to exit the end cap 68while preventing liquid from exiting the end cap 68. The filter 70 may be composed of a plurality of small holes, a mesh, or a mat of fibers. The filter 70 may also be made of a hydrophobic material.

The filter 70 may be sized so that the air flow through the filter 70 is restricted to slow the flow of liquid from the drip chamber 24 and through the tube 60 during priming of the IV set 10. By slowing the flow of liquid, air is less likely to be entrapped within the tube 60 during priming of the IV set 10. Additionally, air is less likely to be pulled from the surface of the liquid, at an operable liquid height 38 in the drip chamber 24, to the outlet orifice 34 and through the tube 60.

To slow the flow of liquid through the tube 60, the IV set 10 may also include a tube attachment device 72 for disposing the second end 64 of the tube 60 proximate to the drip chamber 24. The tube attachment device 72 may be connected to the drip chamber 24, the pressure activated valve 50, near the first end 62 of the tube 60, or to another portion of the IV set 10. The tube attachment device 72 may be integrally formed with a portion of the IV set 10, or may be connected by an adhesive, weld, or mechanical fastener, such as a hook and loop fastener or clip.

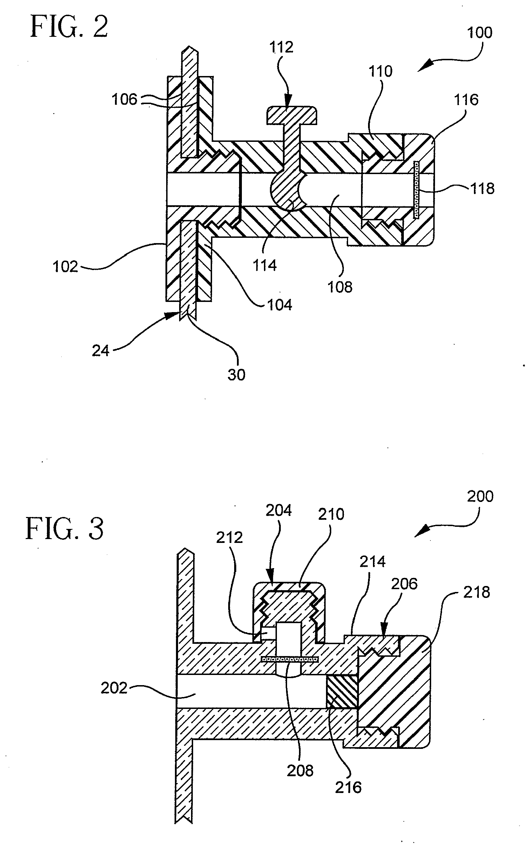

Referring to Figure 2, a cross sectional view illustrates a valve assembly 100 that may be connected to the access orifice 36 in the sidewall 30 of the drip chamber 24. The valve assembly 100 may be clamped to the sidewall 30 at about the access orifice 36 by a male member 102 and a female member 104. The male member 102 and the female member 104 may be threadably attached to permit the valve assembly 100 to be tightly connected to the sidewall 30. Additionally, the male member 102 and female member 104 may be sealed together and to the sidewall 30 by an adhesive 106.

The valve assembly 100 includes a duct 108 that extends from the access orifice 36 to an access port 110 and a valve 112 that controls fluid flow through the duct 108 and thus, the access orifice 36. The valve 112 may also be disposed to control the flow of fluid through the access port 110. As shown, the valve 112 may be a butterfly valve 114.

The access port 110 may be a Luer connector or other connector known in the art. The access port 110 may be connected to a vented cap 116. The vented cap 116 may include a filter 118 that permits air to exit the vented cap 116 while substantially preventing liquid from exiting the vented cap 116. Thus, in some configurations, a small amount of liquid may exit the vented cap through the filter, but is generally prevented from exiting the vented cap 116.

Figure 3 is a cross sectional view of another valve assembly 200 connected to the access orifice 36. As shown, the valve assembly 200 may be integrally formed with the drip chamber 24. The valve assembly 200 may include a passageway 202 that extends from the access orifice 36 and branches separately to a valve 204 and an access port 206. Thus, in this configuration, the valve 204 is not disposed to control the flow of fluid through the access port 206.

The valve assembly 200 may also include a filter 208 that is disposed to substantially prevent liquid from exiting the valve 204. The valve 204 is a slide valve 210 that may be moved to cover a gas orifice 212 extending from the passageway 202.

The access port 206 may be a closed Luer access port 214 that includes a septum 216. The septum 216 helps to prevent inadvertent fluid leakage out of the access port 206. The septum 216 may be pierced to permit fluid flow through the access port 206. As shown, the access port 206 may be connected to a solid end cap 218.

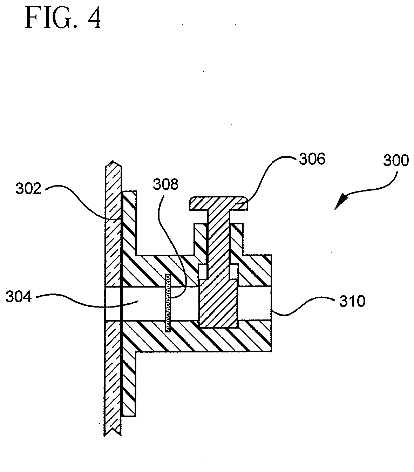

Figure 4 is a cross sectional view of an alternative valve assembly 300 connected to the access orifice 36 of the drip chamber 24 by an adhesive 302. The adhesive 302 also helps to provide a seal between the valve assembly 300 and the drip chamber 24.

The valve assembly 300 may include a passageway 304 extending from the access orifice 36. The valve assembly 300 may also include a valve 306 disposed to control the flow of air through the passageway 304 and a filter 308 disposed to generally prevent liquid from reaching the valve 306. The passageway 304 connects the access orifice 36 with an opening 310.

As shown, the valve 306 may be a slide valve. More specifically, the valve 306 may be slid into the passageway 304 to close the passageway 304 and slid out of the passageway 304 to open the passageway 304.

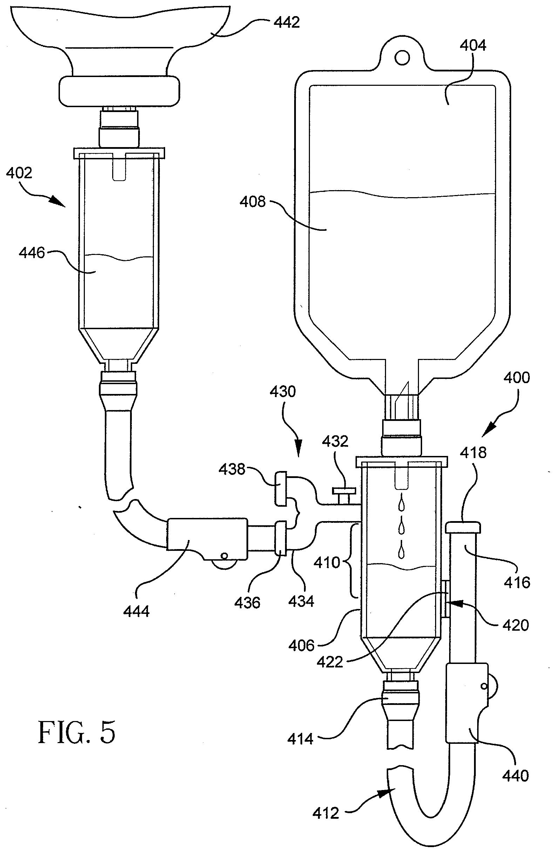

Referring to Figure 5, a side elevation view illustrates an IV set 400 according to the invention and a second IV set 402 piggybacked on the IV set 400. The IV set 400 may be connected to an IV bag 404. Similar to the IV set 10 of Figure 1, the IV set 400 includes a drip chamber 406. The drip chamber 406 may be filled with liquid 408 to an operable liquid height 410. The IV set 400 may also include a tube 412 having a first end 414 connected to the drip chamber 406 and a second end 416 comprising a coupling 418. The second end 416 is disposed proximate the operable liquid height 410 by a tube attachment device 420, which may be a hook and loop fastener 422 with respective portions attached to the drip chamber 406 and the tube 412.

Additionally, the IV set 400 may include a valve assembly 430 attached to the drip chamber 406 near the operable fluid height 410. As shown, the valve assembly 430 may include a valve 432, a manifold 434, a first access port 436, and a second access port 438. Of course, one of skill in the art would recognize that the manifold 434 may provide for more than two access ports 436, 438. The valve 432 may be used to vent air from the drip chamber 406 and may include a filter not shown. The first access port 436 and the second access port 438 may be closed Luer fittings to prevent fluid from leaking out of the access ports 436, 438, while providing access to the drip chamber 406.

The IV set 400 may include a roller clamp 440 for opening and closing the tube 412. The roller clamp 440 may be used to control the flow rate of the liquid 408 in the tube 412.

As shown, the second IV set 402 is coupled to the first access port 436 of the IV set 400 and is connected to an IV bottle 442. The second IV set 402 may include a roller clamp 444 for controlling the flow of liquid 446. The second IV set 402 may be used to deliver medications that are diluted with the liquid 408 of the IV set 400.

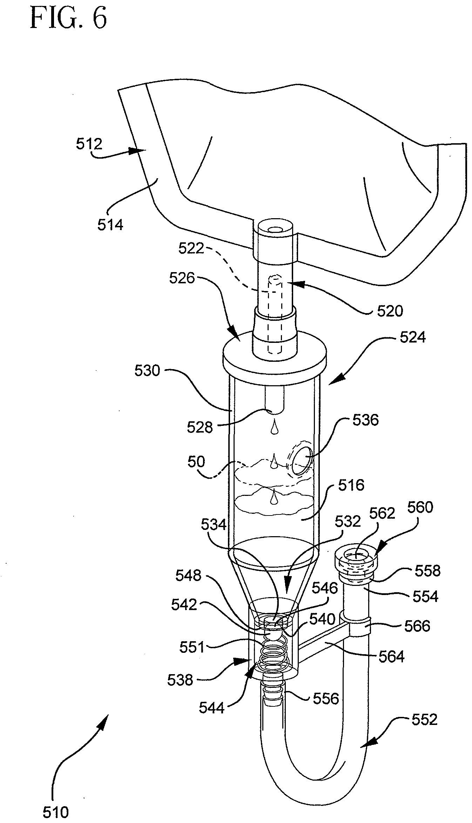

Referring to Figure 6, a perspective view illustrates an IV set 510 connected to a source of liquid 512, such as an IV bag 514 containing a liquid 516, by a coupling 520. The coupling 520 may be a spike 522 that is used to puncture the IV bag 514 and access the liquid 516.

The coupling 520 of the IV set 510 may be connected directly to a drip chamber 524. More specifically, the coupling 520 may be connected to a top end 526 of the drip chamber 524. The top end 526 of the drip chamber 524 may include an inlet orifice 528 that receives liquid 516 from the IV bag 514 through the coupling 520.

The drip chamber 524 may include a sidewall 530 that extends between the top end 526 and a bottom end 532 having an outlet orifice 534. The sidewall 530 may include an air vent 536 that permits air to exit the drip chamber 524 while preventing liquid 516 from exiting the drip chamber 524 through the outlet orifice 534.

The air vent 536 prevents pressure from building in the drip chamber 524 with each droplet of liquid 516 that enters from the IV bag 514 when the flow of liquid 516 through the outlet orifice 534 is blocked. Without the air vent 536, pressure builds within the drip chamber 524 which may cause the flow of liquid 516 to slow until liquid 516 no longer enters the drip chamber 524. Thus, the air vent 536 may be positioned in the sidewall 530 to encourage the liquid 516 to fill to a desired height in the drip chamber 524. Specifically, the liquid 516 is encouraged to enter the drip chamber 524 up to the height of the air vent 536 in the side wall 530. Once the height of the liquid 516 reaches and blocks the air vent 536, the flow of liquid 516 slows and may stop entering the drip chamber 524 until flow through the outlet orifice 534 is unblocked.

To control the flow of liquid 516 through the outlet orifice 534, the outlet orifice 534 may be connected to a pressure activated valve 538. More specifically, the pressure activated valve 538 is disposed so that a sealing orifice 540 is in liquid communication with the outlet orifice 534 of the drip chamber 524. To block the flow of liquid 516 through the outlet orifice 534, a valve 542 may be moved against the sealing orifice 540 by a biasing mechanism 544.

The valve 542 may have a rounded engagement surface 546 that engages and abuts a sharp shoulder 548 of the sealing orifice 540. The engagement surface 546 may be flexible to provide a tight seal against the sealing orifice 540. For example, the valve 542 may be made of an elastomeric material that flexes to seal against the shoulder 548 of the sealing orifice 540.

The pressure activated valve 538 opens and closes in response to the height of liquid 516 being greater than or less than an operable liquid height 550, respectively. Specifically, the biasing mechanism 544 biases the valve 542 against the sealing orifice 540 with a force equal to about the head between the sealing orifice 540 and an operable liquid height 550 in the drip chamber 524 when the drip chamber 524 is held vertically. Thus, as the height of liquid 516 in the drip chamber 524 exceeds the operable liquid height 550, the pressure of the liquid 516 exceeds the biasing force of the biasing mechanism 544 to move the valve 542 away from the sealing orifice 540 and permit the flow of liquid 516 through the outlet orifice 534 of the drip chamber 524 and the pressure activated valve 538. When the height of liquid 516 in the drip chamber 524 is less than the operable liquid height 550, the pressure of the liquid 516 is less than the biasing force of the biasing mechanism 544. Therefore, the valve 542 engages and seals against the sealing orifice 540 preventing the flow of liquid 516 through the outlet orifice 534 of the drip chamber 524 and the pressure activated valve 538.

The biasing mechanism 544 in this configuration of the invention may include a spring 551. When the pressure activated valve 538 is assembled, the spring 551 may be attached to the valve 542 and compressed to store potential energy and bias the valve 542 against the sealing orifice 540.

Alternatively, the biasing mechanism 544 may include a portion of the valve 542 that is buoyant. Additionally, the spring 551 may tend to pull the valve 542 away from the sealing orifice 540. However, in this configuration, the biasing mechanism 544 is able to bias the valve 542 against the sealing orifice 540 because the buoyancy force of the portion of the valve 542 is greater than the force of the spring 551.

The pressure activated valve 538 may be connected to a tube 552 for conveying the flow of liquid 516 from the drip chamber 524. Specifically, the tube 552 may have a first end 554 and a second end 556 that may be connected to the pressure activated valve 538.

The first end 554 may have a connector 558 for connecting the IV set 510 to a patient (not shown), another IV set (not shown) in a piggy back arrangement, or another device known in the art. For example, the connector 558 may be connected to an end plug 560 that may be solid or include an air vent 562 that permits air to exit the tube 552 but prevents liquid 516 from exiting the first end 554 of the tube 552. The air vent 562 may also be designed to restrict the flow of air out of the first end 554 of the tube 552 in order to slow the flow of liquid 516 that moves through the IV set 510 when the IV set 510 is primed. The connector 558 may be Luer fitting for a threaded or press fit connection or another connector known in the art.

The IV set 510 may also include a tube attachment device 564 for disposing the second end 556 of the tube 552 proximate to the drip chamber 524 during priming of the IV set 510. Disposing the second end 556 of the tube 552 proximate to the drip chamber 524 during priming slows the flow of liquid 516 through the IV set 510 via the hydrostatic pressure of the liquid 516. As shown, the tube attachment device 564 may be integrally formed with the pressure activated valve 538 and may include a clip 566 for removable attachment of the tube 552 to the tube attachment device 564.

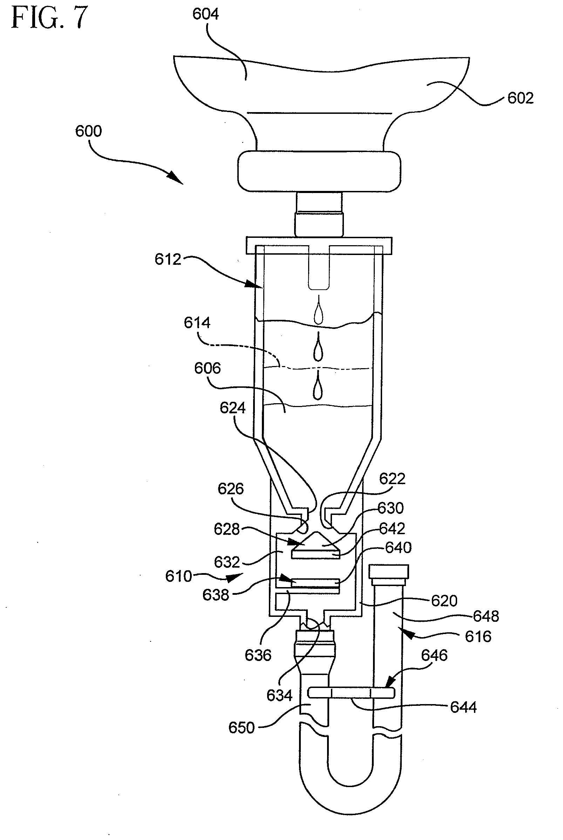

Referring to Figure 7, a partially cut away side view illustrates another IV set 600 that may be connected to a source of liquid 602, such as an IV bottle 604 containing a liquid 606. The IV set 600 includes many features similar to the IV set 10 of Figure 1. For brevity similar features may not be discussed in detail.

As shown, a pressure activated valve 610 is open because the height of the liquid 606 in a drip chamber 612 exceeds the operable liquid height 614 of the drip chamber 612. Thus, the liquid 606 is able to flow from the drip chamber 612 through the pressure activated valve 610 to a tube 616.

The pressure activated valve 610 may include a housing 620 that defines a sealing orifice 622 in liquid communication with an outlet orifice 624 of the drip chamber 612. The sealing orifice 622 may include a beveled shoulder 626 that may be engaged by a valve 628 to close the sealing orifice 622. The valve 628 may have an engagement surface 630 that is reciprocally shaped to the shape of the sealing orifice 622 to facilitate engaging and closing the sealing orifice 622. Thus, the engagement surface 630 may be beveled.

The housing 620 further defines a flow chamber 632 that extends from the sealing orifice 622 to an exit orifice 634 that may be connected to the tube 616. The housing 620 may also include a support feature 636 for positioning a biasing mechanism 638 within the flow chamber 632. Specifically, the support feature 636 may extend into the flow chamber 632 to position the biasing mechanism 638 in line with the sealing orifice 622.

The biasing mechanism 638 may include a base magnet 640 that repels a valve magnet 642. The valve magnet 642 is a part of the valve 628 and attached to the engagement surface 630. The pressure activated valve 610 may be opened when the pressure of the liquid 606 exceeds the magnetic force between the opposed base magnet 640 and the valve magnet 642.

The IV set 600 may also include a tube attachment device 644 that may include two clips 646 for gripping a first end 648 and a second end 650 of the tube 616. The tube attachment device 644 may be used to position the first end 648 of the tube 616 near the drip chamber 612.

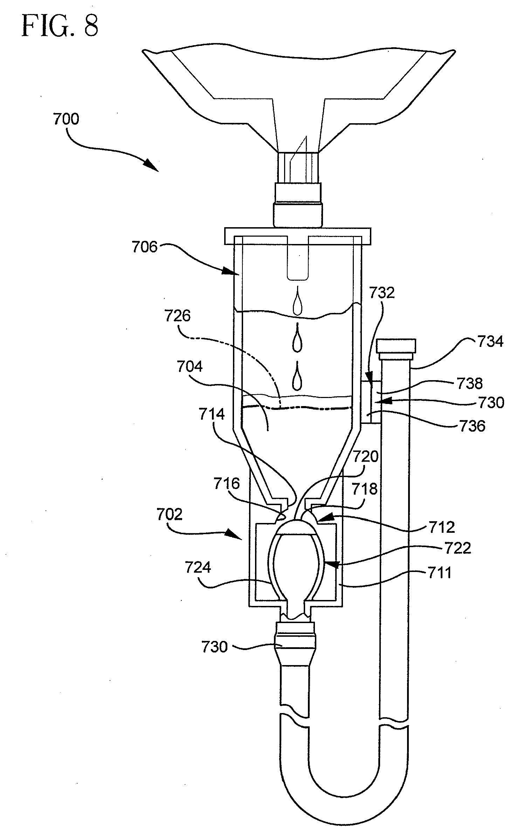

Figure 8 is a partially cut away side view showing an alternative IV set 700. The IV set 700 includes many features similar to the IV set 10 of Figure 1. For brevity, similar features may not be discussed in detail.

As shown, a pressure activated valve 702 is open because the height of the liquid 704 in the drip chamber 706 exceeds the operable liquid height 708. Because the pressure activated valve 702 is open, liquid 704 may flow from a drip chamber 706 and pass through the pressure activated valve 702 into a tube 710.

The pressure activated valve 702 may have a housing 711 that includes a sealing orifice 712 that is in liquid communication with an outlet orifice 714 of the drip chamber 706. The sealing orifice 712 may have a curved shoulder 716 that mates with a curved engagement surface 718 of a valve 720 of the pressure activated valve 702. The valve 720 is positioned and supported by a biasing mechanism 722.

The biasing mechanism 722 comprises arms 724 that may be attached to the housing 711. The arms 724 may be flexible so that the arms 724 bias the valve 720 toward the sealing orifice 712. The flexible arms 724 may be similar to a spring that stores potential energy as they flex in response to the head between the sealing orifice 712 and the height of the liquid 704 in the drip chamber 706, such that the valve 720 may move away from the sealing orifice 712 when the height of the liquid 704 exceeds the operable liquid height 726 of the drip chamber 706.

The IV set 700 may also include a tube attachment device 730. The tube attachment device may include a hook and loop fastener 732 for disposing a first end 734 of the tube 710 proximate to the drip chamber 706. As shown, a fabric strip of hooks 736 may be attached to the drip chamber 706 and a fabric strip of loops 738 may be connected to the first end 734 of the tube 710 to facilitate the positioning of the first end 734 of the tube 710 near the drip chamber 706.

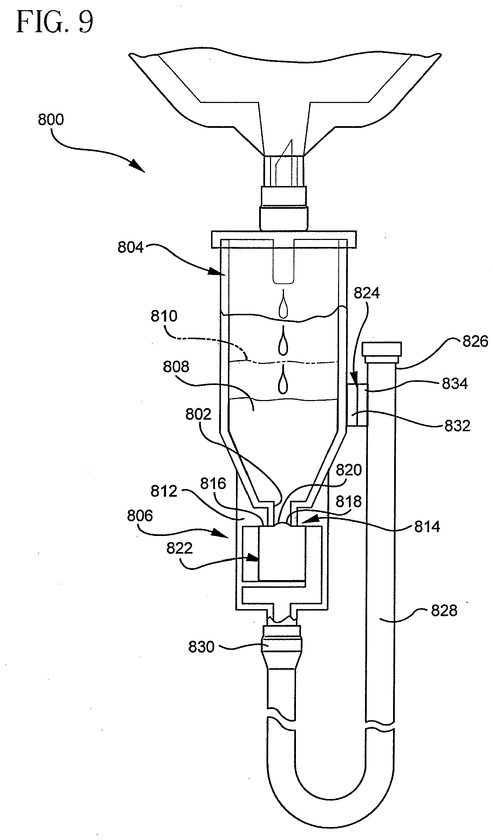

Figure 9 is a partially cut away side view illustrating yet another IV set 800. The IV set 800 includes many features similar to the IV set 10 of Figure 1. For brevity, similar features may not be discussed in detail.

As shown in Figure 9, an outlet orifice 802 of a drip chamber 804 is connected to a pressure activated valve 806. The pressure activated valve 806 is closed, because the height of a liquid 808 in the drip chamber 804 has not equaled or exceeded the operable liquid height 810. Therefore, the liquid 808 is unable to flow through the outlet orifice 802 of the drip chamber 804.

The pressure activated valve 806 includes a housing 812 that includes a sealing orifice 814 that has a sharp shoulder 816. An engagement surface 818 of a valve 820 may engage the shoulder 816 and close the sealing orifice 814.

As shown, the valve 820 may be integrally formed with a biasing mechanism 822. The valve 820 and biasing mechanism 822 may be an arm of elastomeric material that extends between the sealing orifice 814 and a support feature 824 of the housing 812. In some configurations, the valve 820 and biasing mechanism 822 may be made of a closed cell, foamed material or a solid material. Additionally, the valve 820 and biasing mechanism 822 may be made of an elastomer or other material known in the art.

As the height of the liquid 808 increases in the drip chamber 804, the biasing mechanism 822 compresses under the pressure of the liquid 808 in the drip chamber. When the liquid 808 is about equal to or exceeds the operable liquid height 810, the engagement surface 818 disengages from the sealing orifice 814 and the liquid 808 is able to flow through the outlet orifice 802 of the drip chamber 804 and the housing 812 of the pressure activated valve 806. Once the height of the liquid 808 falls below the operable liquid height 810, the biasing mechanism 822 expands to force the engagement surface 818 against the shoulder 816 of the sealing orifice 814 and close the pressure activated valve 806.

The IV set 800 may also include a tube attachment device 824 for disposing a first end 826 of a tube 828 near the drip chamber 804. A second end 830 of the tube 828 may be connected to the pressure activated valve 806. The tube attachment device 824 may include a first magnet 832 attached to the drip chamber 804 and a second magnet 834 attached to the first end 826 of the tube 828. Thus, to position the first end 826 of the tube 828, the first magnet 832 may be brought near the second magnet 834.

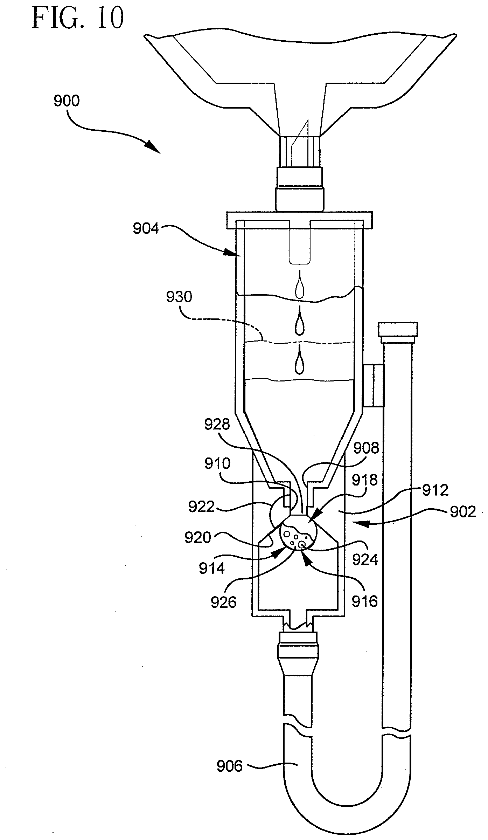

In reference to Figure 10, a partially cut away side view illustrates a different IV set 900 according to the invention. The IV set 900 is similar in many ways to the IV set 800 of Figure 9. Thus, for brevity, only the differences will de discussed detail. As shown, the IV set 900 includes a pressure activated valve 902 disposed between a drip chamber 904 and the tubing 906 of the IV set 900. Specifically, an outlet orifice 908 of the drip chamber 904 is connected to a sealing orifice 910 of a housing 912 of the pressure activated valve 902. The housing 912 of the pressure activated valve 902 also includes an exit orifice 910 that is connected to the tubing 906.

The housing 912 contains a structure 914 that includes the biasing mechanism 916 and valve 918 of the pressure activated valve 902. The housing 912 includes a wall 920 that is disposed at an angle 922 to the sealing orifice 910. The angle 922 of the wall 920 guides the valve 918 as it moves within the housing 912 to seat against the sealing orifice 910 and close the pressure activated valve 902.

The biasing mechanism 916 of the structure 914 is total volume of the structure 914 having an average density less than water. As shown in a partially cut away view, the average density less than water may be obtained by including one or more air bubbles 924 within the structure 914, by making the structure 914 out of a material 926 whose density is less than the density of water, or a combination of these methods. The valve 918 is the outer surface 928 of the structure 914 that contacts and seats against the sealing orifice 910 to close the pressure activated valve 902.

The pressure activated valve 902 operates by initially allowing fluid to pass through the pressure activated valve 902. As the housing 912 fills with liquid, the structure 914 floats upward to seat against the sealing orifice 910 and close the pressure activated valve 902. Thus, the valve 918 is held against the sealing orifice 910 by the buoyancy force of the biasing mechanism 916.

Once the liquid level in the drip chamber 904 reaches an operable fluid height 930, the valve 918 is forced away from the sealing orifice 910 to open the pressure activated valve 902. Specifically, the pressure head between sealing orifice 910 and the operable fluid height 930 is greater than the buoyancy force of the biasing mechanism 916, which opens the pressure activated valve 902.

In summary, an IV set according to the invention may include a pressure activated valve that opens when an operable liquid height is reached and closes when the liquid in the drip chamber falls below the operable liquid height. The operable liquid height is the height of liquid in the drip chamber that exerts pressure on the valve about equal to the force exerted by the biasing mechanism on the valve against the sealing orifice.

The flow of the liquid may be retarded by using an air filter and positioning the first end of the tube near the drip chamber. A slow moving liquid may be less likely to entrap air in the IV set. Thus, less air may be entrapped and less time needed to remove air bubbles from the IV set before it may be connected to a patient.

The present invention may be embodied in other specific forms without departing from its structures, methods, or other essential characteristics as broadly described herein and claimed hereinafter. The described embodiments are to be considered in all respects only as illustrative, and not restrictive. The scope of the invention is, therefore, indicated by the appended claims, rather than by the foregoing description. All changes that come within the meaning and range of equivalency of the claims are to be embraced within their scope.

## Claims

1. An IV set comprising:
a drip chamber (24) comprising an access orifice (36), a top end (26) having an inlet orifice (32), a bottom end (28) having an outlet orifice (34), and a sidewall extending between the top end (26) and the bottom end (28), wherein the access orifice (36) is disposed in the sidewall of the drip chamber (24);
a coupling (20) for connecting the IV set to a source of liquid (12); and
a valve assembly (100) connected to the access orifice (36), the valve assembly (100) comprising:
a valve (46) connected to the drip chamber (24) for selectively controlling access to the drip chamber (24) through the access orifice (36);
an access port (110);
a duct (108) that extends from the access orifice (36) to the access port (110);
**characterised in that** the valve assembly (100) further comprises a vented cap (116) connected to the access port (110),

2. The IV set of claim 1, further comprising a filter (48) connected to the access orifice (36) of the drip chamber (24) for permitting air to flow through the filter (48) while restricting the flow of liquid.

3. The IV set of claim 1, wherein the valve (46) is disposed to control the flow of fluid through the access port (40).

4. The IV set of claim 1, wherein the access port (40) comprises a septum (216).

5. The IV set of claim 1, wherein the access orifice (36) is disposed in the sidewall at or proximate to an operable liquid height (38).

6. The IV set of claim 1, further comprising a pressure activated valve (50) disposed to control the flow of liquid through the outlet orifice (34) of the drip chamber (24).

7. The IV set of claim 1, further comprising a tube (60) having a first end (62) connected to the drip chamber (24) and a second end (64), wherein the IV set further comprises a tube attachment device (72) for disposing the second end (64) of the tube proximate to the drip chamber.

8. The IV set of claim 1, further comprising a manifold (434) connected to the access orifice (36).

9. The IV set of claim 1, further comprising:
a tube in liquid communication with the outlet orifice of the drip chamber (24); and
a pressure activated valve (50) disposed proximate the outlet orifice (34) of the drip chamber (24).

10. The IV set of claim 9, wherein the pressure activated valve (50) further comprises a sealing orifice, a valve (54, 642), and a biasing member (56) that biases the valve against the sealing orifice (622) with a force that is less than the pressure head between the sealing orifice (622) and an operable liquid height (38) in the drip chamber (24) to control the flow of the liquid through the pressure activate valve (50).

11. The IV set of claim 10, further comprising a filter (48) connected to the access orifice (36) of the drip chamber (24) for permitting air to flow through the filter (48) while restricting the flow of liquid.

12. The IV set of claim 11, further comprising an access port (40) connected to the access orifice (36) of the drip chamber (24).

13. The IV set of claim 11, wherein the access orifice (36) is connected to the sidewall at or proximate to an operable liquid height (38).

14. The IV set of claim 9, further comprising a manifold (434) connected to the access orifice (36).

## Patentansprüche

1. IV-Set, umfassend:
eine Tropfkammer (24), die eine Zugangsöffnung (36),
ein oberes Ende (26) mit einer Einlassöffnung (32),
ein unteres Ende (28) mit einer Auslassöffnung (34) und eine sich zwischen dem oberen Ende (26) und dem unteren Ende (28) erstreckende Seitenwand umfasst,
wobei die Zugangsöffnung (36) in der Seitenwand der Tropfkammer (24) angeordnet ist,
eine Koppelung (20) zum Verbinden des IV-Sets mit einer Flüssigkeitsquelle (12) und
einer Ventilanordnung (100), die mit der Zugangsöffnung (36) verbunden ist, wobei die Ventilanordnung (100) Folgendes umfasst:
ein Ventil (46), das mit der Tropfkammer (24) verbunden ist, um den Zugang zu der Tropfkammer (24) durch die Zugangsöffnung (36) gezielt zu steuern,
einen Zugangsanschluss (110),
einen Kanal (108), der sich von der Zugangsöffnung (36) zu dem Zugangsanschluss (110) erstreckt,
**dadurch gekennzeichnet, dass** die Ventilanordnung (100) ferner eine mit dem Zugangsanschluss (110) verbundene belüftete Kappe (116) umfasst.

2. IV-Set nach Anspruch 1, ferner umfassend einen Filter (48), der mit der Zugangsöffnung (36) der Tropfkammer (24) verbunden ist, damit Luft durch den Filter (48) strömen kann, während die Strömung von Flüssigkeit eingeschränkt wird.

3. IV-Set nach Anspruch 1, wobei das Ventil (46) dazu angeordnet ist, die Strömung von Flüssigkeit durch den Zugangsanschluss (40) zu steuern.

4. IV-Set nach Anspruch 1, wobei der Zugangsanschluss (40) ein Septum (216) umfasst.

5. IV-Set nach Anspruch 1, wobei die Zugangsöffnung (36) an einem oder in der Nähe eines Betriebsflüssigkeitsstands (38) in der Seitenwand angeordnet ist.

6. IV-Set nach Anspruch 1, ferner umfassend ein druckaktiviertes Ventil (50), das dazu angeordnet ist, die Strömung von Flüssigkeit durch die Auslassöffnung (34) der Tropfkammer (24) zu steuern.

7. IV-Set nach Anspruch 1, ferner umfassend eine Röhre (60) mit einem ersten Ende (62), das mit der Tropfkammer (24) verbunden ist, und einem zweiten Ende (64), wobei das IV-Set ferner eine Röhrenanbringvorrichtung (72) umfasst, um das zweite Ende (64) der Röhre in der Nähe der Tropfkammer anzuordnen.

8. IV-Set nach Anspruch 1, ferner umfassend einen Verteiler (434), der mit der Zugangsöffnung (36) verbunden ist.

9. IV-Set nach Anspruch 1, ferner umfassend:
eine Röhre in flüssiger Kommunikation mit der Auslassöffnung der Tropfkammer (24) und
ein druckaktiviertes Ventil (50), das in der Nähe der Auslassöffnung (34) der Tropfkammer (24) angeordnet ist.

10. IV-Set nach Anspruch 9, wobei das druckaktivierte Ventil (50) ferner eine Dichtöffnung, ein Ventil (54, 642) und ein Vorspannglied (56) umfasst, das das Ventil mit einer Kraft gegen die Dichtöffnung (622) vorspannt, die geringer als die Druckhöhe zwischen der Dichtöffnung (622) und einem Betriebsflüssigkeitsstand (38) in der Tropfkammer (24) ist, um die Strömung der Flüssigkeit durch das druckaktivierte Ventil (50) zu steuern.

11. IV-Set nach Anspruch 10, ferner umfassend einen Filter (48), der mit der Zugangsöffnung (36) der Tropfkammer (24) verbunden ist, damit Luft durch den Filter (48) strömen kann, während die Strömung von Flüssigkeit eingeschränkt wird.

12. IV-Set nach Anspruch 11, ferner umfassend einen Zugangsanschluss (40), der mit der Zugangsöffnung (36) der Tropfkammer (24) verbunden ist.

13. IV-Set nach Anspruch 11, wobei die Zugangsöffnung (36) an einem oder in der Nähe eines Betriebsflüssigkeitsstands (38) mit der Seitenwand verbunden ist.

14. IV-Set nach Anspruch 9, ferner umfassend einen Verteiler (434), der mit der Zugangsöffnung (36) verbunden ist.

## Revendications

1. Nécessaire à perfusion comprenant :
une chambre compte-gouttes (24), comprenant :
un orifice d'accès (36) ;
une extrémité supérieure (26) comportant un orifice d'entrée (32) ;
une extrémité inférieure (28) comportant un orifice de sortie (34) ; et
une paroi latérale s'étendant entre l'extrémité supérieure (26) et l'extrémité inférieure (28),
dans lequel l'orifice d'accès (36) est disposé dans la paroi latérale de la chambre compte-gouttes (24) ;
un raccord (20) pour raccorder le nécessaire à perfusion à une source de liquide (12) ; et
un ensemble robinet (100) raccordé à l'orifice d'accès (36), l'ensemble robinet (100) comprenant :
un robinet (46) raccordé à la chambre compte-gouttes (24) pour commander sélectivement l'accès à la chambre compte-gouttes (24) par l'orifice d'accès (36) ;
un orifice d'accès (110) ;
un conduit (108) qui s'étend de l'orifice d'accès (36) à l'orifice d'accès (110),
**caractérisé en ce que** l'ensemble robinet (100) comprend en outre un bouchon à évent (116) raccordé à l'orifice d'accès (110).

2. Nécessaire à perfusion selon la revendication 1, comprenant en outre un filtre (48) raccordé à l'orifice d'accès (36) de la chambre compte-gouttes (24) pour permettre à l'air de circuler par le filtre (48) tout en limitant l'écoulement de liquide.

3. Nécessaire à perfusion selon la revendication 1, dans lequel le robinet (46) est disposé pour commander l'écoulement de fluide par l'orifice d'accès (40).

4. Nécessaire à perfusion selon la revendication 1, dans lequel l'orifice d'accès (40) comprend un septum (216).

5. Nécessaire à perfusion selon la revendication 1, dans lequel l'orifice d'accès (36) est disposé dans la paroi latérale au niveau ou à proximité d'une hauteur de liquide (38) apte au fonctionnement.

6. Nécessaire à perfusion selon la revendication 1, comprenant en outre une valve (50) actionnée par pression disposée pour commander l'écoulement de liquide par l'orifice de sortie (34) de la chambre compte-gouttes (24).

7. Nécessaire à perfusion selon la revendication 1, comprenant en outre un tube (60) comportant une première extrémité (62) raccordée à la chambre compte-gouttes (24) et une seconde extrémité (64),
dans lequel le nécessaire à perfusion comprend en outre un dispositif (72) de fixation de tube pour disposer la seconde extrémité (64) du tube à proximité de la chambre compte-gouttes.

8. Nécessaire à perfusion selon la revendication 1, comprenant en outre un collecteur (434) raccordé à l'orifice d'accès (36).

9. Nécessaire à perfusion selon la revendication 1, comprenant en outre :
un tube en communication liquide avec l'orifice de sortie de la chambre compte-gouttes (24) ; et
une valve (50) actionnée par pression disposée à proximité de l'orifice de sortie (34) de la chambre compte-gouttes (24).

10. Nécessaire à perfusion selon la revendication 9, dans lequel la valve (50) actionnée par pression comprend en outre un orifice d'étanchéité, un clapet (54, 642) et un élément de sollicitation (56) qui sollicite le clapet contre l'orifice d'étanchéité (622), avec une force qui est inférieure à la hauteur manométrique entre l'orifice d'étanchéité (622) et un niveau (38) de liquide apte au fonctionnement dans la chambre compte-gouttes (24), pour commander l'écoulement du liquide par la valve (50) actionnée par pression.

11. Nécessaire à perfusion selon la revendication 10, comprenant en outre un filtre (48) raccordé à l'orifice d'accès (36) de la chambre compte-gouttes (24) pour permettre à l'air de circuler par le filtre (48) tout en limitant l'écoulement de liquide.

12. Nécessaire à perfusion selon la revendication 11, comprenant en outre un orifice d'accès (40) raccordé à l'orifice d'accès (36) de la chambre compte-gouttes (24).

13. Nécessaire à perfusion selon la revendication 11, dans lequel l'orifice d'accès (36) est raccordé à la paroi latérale au niveau ou à proximité de la hauteur de liquide (38) apte au fonctionnement.

14. Nécessaire à perfusion selon la revendication 9, comprenant en outre un collecteur (434) raccordé à l'orifice d'accès (36).
